Europäisches Patentamt

European Patent Office

Office européen des brevets

(11) Publication number: **0 268 452 B1**

(12) **EUROPEAN PATENT SPECIFICATION**

(45) Date of publication of patent specification: **22.01.92**

(51) Int. Cl.⁵: **C12N 9/18**, C12N 15/55, C12N 1/20, //(C12N1/20, C12R1:40)

(21) Application number: **87310120.8**

(22) Date of filing: **17.11.87**

(54) **Novel hydrolase and method of production.**

(30) Priority: **19.11.86 US 932959**
**19.10.87 US 107902**

(43) Date of publication of application:
**25.05.88 Bulletin  88/21**

(45) Publication of the grant of the patent:
**22.01.92 Bulletin  92/04**

(84) Designated Contracting States:
**DE FR GB**

(56) References cited:
**EP-A- 0 253 487**
**JP-A-60 188 072**

CHEMICAL ABSTRACTS, vol. 89, 1978, pages 318-319, abstract no. 19964b, Columbus, Ohio, US; N.A. BASHKATOVA et al.: "Exolipases of certain pseudomonas species", & MIKROBIOLOGIYA 1978, 47(2), 234-40

(73) Proprietor: **GENENCOR, INC.**
**180 Kimball Way**
**South San Francisco California 94080(US)**

(72) Inventor: **Gray, Gregory L.**
**530 Elm Court**
**South San Francisco California 94080(US)**
Inventor: **Poulose, Ayrookaram J.**
**2540 Carmel Drive**
**San Bruno California 94066(US)**
Inventor: **Power, Scott D.**
**732 Olive Court**
**San Bruno California 94066(US)**

(74) Representative: **Armitage, Ian Michael et al**
**MEWBURN ELLIS & CO. 2/3 Cursitor Street**
**London EC4A 1BO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention generally relates to enzymes, and more particularly relates to a novel hydrolase, isolatable from pseudomonas putida ATCC 53552 and purifiable as a substantially enzymatically pure peptide, and a method for producing the hydrolase by cloning.

Psudomonas is a genus of short, rod-shaped bacteria. Several strains, including P. putida, have been shown to have a limited ability to grow on a minimal media with mono-oleate polyoxyethylene ("Tween 80", available from Atlas Chemical) as carbon source. Howe et al., J. Gen. Microbiol., 92(1), pp. 234-235 (1976). Various uses have been described for strains belonging to the genus Psuedomonas.

U.S. Patent 4,385,112, issued May 24, 1983, inventors Misaki et al., discloses use of a microorganism strain belonging to the genus Pseudomonas isolated from a soil sample from an onion field in Japan to produce a nucleoside oxidase useful for enzymatic reactions involving various nucleosides. U.S. patent 4,430,433, issued February 7, 1984, inventors Hammond et al., discloses use of the Pseudomonas putida strain to produce aryl acyl amidase enzymes (which catalyze the hydrolysis of anilides to anilines plus fatty acid anions) of molecular weight between about 48,000 and 60,000. These aryl acyl amidases are said to be useful in methods of analyzing N-acylated primary aromatic amines.

U.S. Patent 4,542,100, issued September 17, 1985, inventor Hagedorn, discloses a process for producing p-cresol in conjunction with a Pseudomonas putida strain. U.S. Patent 4,588,688, issued May 13, 1986, inventor Maxwell, discloses a process for the production of muconic acid in a bioconversion medium with a Pseudomonas putida strain. Linden and Benisek have described an isomerase from Pseudomonas putida biotype B. J. Biol. Chem. 261, No. 14, pp. 6454-6560 (May 15, 1986).

In short, novel strains of Pseudomonas putida producing various enzymes have recently been discovered for a variety of applications.

In one aspect of the present invention, a novel enzyme is provided with hydrolase activity. This hydrolase is secreted by Pseudomonas putida ATCC 53552 (now thought to be better classified as P. mendocino) and is isolatable as a substantially enzymatically pure peptide. In another aspect of the present invention, the novel hydrolase is produced in high yield by cloning the gene expressing the hydrolase into a suitable expression vector, and culturing to express the gene for the hydrolase.

The novel hydrolase is useful in a variety of applications, such as in biomass processing for breakdown of cellular materials (e.g. cutin and the like) and in enzymatic perhydrolysis for laundry and bleaching.

In the drawings:

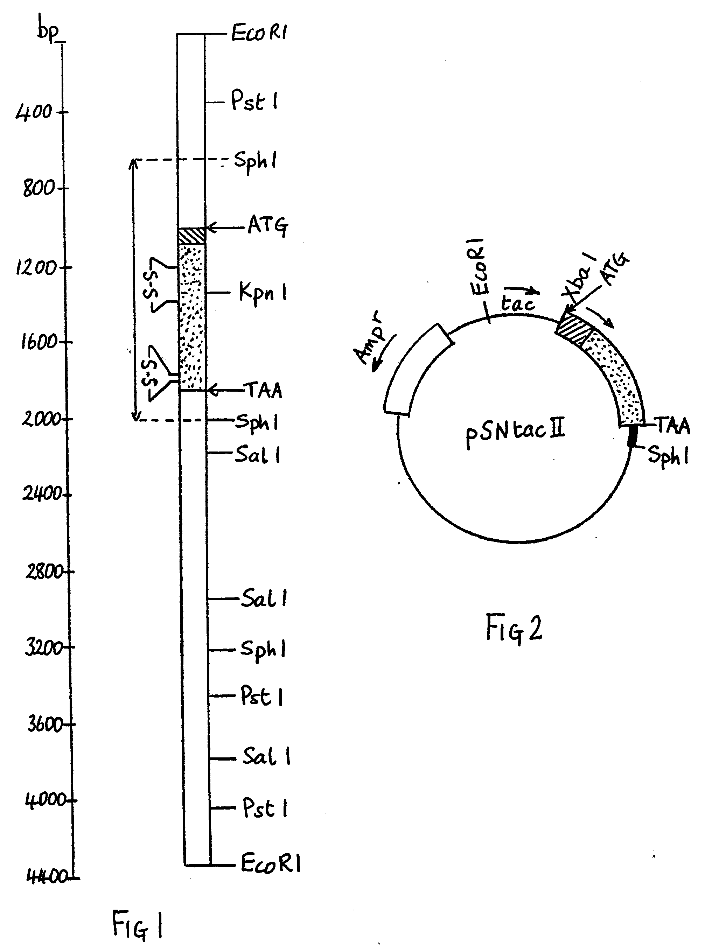

Figure 1 is a map of the 4.3 kb EcoRI fragment of a plasmid designated pSNE4. The region crosshatched represents signal peptide codons (codons -22 to +1) and the stippled region indicates the coding region (codons +1 to +258) for the mature polypeptide designated Hydrolase 1. The ATG initiation codon and TAA stop codon are also marked; and

Figure 2 illustrates an E. coli expression vector for Pseudomonas Hydrolase 1 gene. The crosshatched region indicates the coding region for the hydrolase signal sequence of 22 amino acids. The stippled region indicates the coding region for the mature hydrolase protein. Transcription starts at the ATG initiation codon and proceeds in the direction indicated by the arrow to the TAA stop codon. The dark regions on either side indicate the 5'- and 3-'untranslated regions.

Description of the Preferred Embodiments

In order to ensure proper understanding and interpretation of the invention, including the summary and preferred embodiments as well as the claims, some definitions are set forth below to clarify the use of terms employed herein. The defined terms include the following.

"Perhydrolysis" is defined as the reaction of a selected substrate with peroxide to form a peracid and water.

"Enzymatic perhydrolysis" is defined as a perhydrolysis reaction which is assisted or catalyzed by an enzyme generally classified as a hydrolase, and more specifically identified below.

The novel enzyme (sometimes herein referred to as "Hydrolase 1") is secreted by and isolatable from Pseudomonas. A culture of a novel Pseudomonas mendocino (formerly P. putida) strain from which the Hydrolase 1 enzyme may be isolated is deposited in accordance with the Budapest Treaty in the permanent culture collection of the American Type culture Collection, 12301 Parklawn Drive, Rockville, Maryland 20852, and has been designated ATCC 53552.

It should be understood that the microorganism of the present invention is not limited to the Pseudomonas mendocino strain hereinabove described, as natural and artificial mutants of the said microorganism can be used. Mutant or variant strains of Pseudomonas mendocino ATCC 53522 may be obtained by environmental selection pressure techniques, by UV irradiation, or by the use of mutagenic chemicals. As described hereinafter, genetic engineering techniques applicable to hydrolase production, such as transformation of corresponding genes of the present strain to other cells, are preferably applied for commercial production of the hydrolase.

However, the Pseudomonas mendocino strain may be cultured in a conventional medium. Liquid or solid culture can be used. Submerged aeration culture is preferable. A conventional nutrient medium can be used. Culturing temperature may vary depending on the desired rate of growth of the microorganisms and is preferably at 25°-35°C. Culturing time can be selected as desired, and is 15-50 hours. Culturing may be terminated when the highest concentration of hydrolase is present in the medium.

Hydrolase is accumulated in the fermentation broth, and extraction of the produced enzyme from the broth can be effected as follows. Cells and cell debris are first removed from the whole cell broth culture by microfiltration and centrifugation, followed by ultrafiltration to concentrate the hydrolase. Excess salts and color are then removed by dialysis or diafiltration.

The crude enzyme solution can then be purified. A powder of the enzyme can be obtained by lyophilization and used in the various applications.

The Hydrolase 1 and its encoding DNA have the following amino acid sequence:

```
                                    1                               10
                          ala pro leu pro asp thr pro gly ala pro
                          GCT CCC CTG CCG GAT ACA CCG GGA GCG CCA


                                   20                               30
phe pro ala val ala asn phe asp arg ser gly pro tyr thr thr ser ser gln ser glu
TTT CCG GCT GTC GCC AAT TTC GAC CGC AGT GCC CCC TAC ACC ACC AGC AGC CAG AGC GAG


                                   40                               50
gly pro ser cys arg ile tyr arg pro arg asp leu gly gln gly gly val arg his pro
GGG CCG AGC TGT CGC ATC TAT CGG CCC CGC GAC CTG GGT CAG GGG GGC GTG CGT CAT CCG


                                   60                               70
val ile leu trp gly asn gly thr gly ala gly pro ser thr tyr ala gly leu leu ser
GTG ATT CTC TGG GGC AAT GGC ACC GGT GCC GGG CCG TCC ACC TAT GCC GGC TTG CTA TCG


                                   80                               90
his trp ala ser his gly phe val val ala ala ala glu thr ser asn ala gly thr gly
CAC TGG GCA AGC CAC GGT TTC GTG GTG GCG GCG GCG GAA ACC TCC AAT GCC GGT ACC GGG


                                  100                              110
arg glu met leu ala cys leu asp tyr leu val arg glu asn asp thr pro tyr gly thr
CGG GAA ATG CTC GCC TGC CTG GAC TAT CTG GTA CGT GAG AAC GAC ACC CCC TAC GGC ACC


                                  120                              130
tyr ser gly lys leu asn thr gly arg val gly thr ser gly his ser gln gly gly gly
TAT TCC GGC AAG CTC AAT ACC GGG CGA GTC GGC ACT TCT GGG CAT TCC CAG GGT GGT GGC


                                  140                              150
gly ser ile met ala gly gln asp thr arg val arg thr thr ala pro ile gln pro tyr
GGC TCG ATC ATG GCC GGG CAG GAT ACG AGG GTG CGT ACC ACG GCG CCG ATC CAG CCC TAC


                                  160                              170
thr leu gly leu gly his asp ser ala ser gln arg arg gln gln gly pro met phe leu
ACC CTC GGC CTG GGG CAC GAC AGC GCC TCG CAG CGG CGG CAG CAG GGG CCG ATG TTC CTG


                                  180                              190
met ser gly gly gly asp thr ile ala phe pro tyr leu asn ala gln pro val tyr arg
ATG TCC GGT GGC GGT GAC ACC ATC GCC TTT CCC TAC CTC AAC GCT CAG CCG GTC TAC CGG


                                  200                              210
arg ala asn val pro val phe trp gly glu arg arg tyr val ser his phe glu pro val
CGT GCC AAT GTG CCG GTG TTC TGG GGC GAA CGG CGT TAC GTC AGC CAC TTC GAG CCG GTC


                                  220                              230
gly ser gly gly ala tyr arg gly pro ser thr ala trp phe arg phe gln leu met asp
GGT AGC GGT GGG GCC TAT CGC GGC CCG AGC ACG GCA TGG TTC CGC TTC CAG CTG ATG GAT


                                  240                              250
asp gln asp ala arg ala thr phe tyr gly ala gln cys ser leu cys thr ser leu leu
GAC CAA GAC GCC CGC GCT ACC TTC TAC GGC GCG CAG TGC AGT CTG TGC ACC AGC CTG CTG


         258
trp ser val glu arg arg gly leu
TGG TCG GTC GAG CGC CGC GGG CTT
```

Preferably, the hydrolase is produced by genetic manipulation techniques, for example by the transfer of plasmid DNA to a multicopy host or by the excision of the chromosomal genes coding for the hydrolase from the cells of a hydrolase producing bacteria, followed by the cloning of said genes into a suitable vector molecule. A preferred means of producing Hydrolase 1 is by cloning.

Thus, and as further described in Example 9, Figure 1 is a map of the 4.3 kb EcoRI fragment of pSNE4. The crosshatched box represents the signal peptide codons (codons -22 to +1), and the stippled region indicates the coding region for the mature Hydrolase 1 polypeptide codons +1 to +258. The postulated disulfide bonds are shown. The scale is in base pairs (bp). The region sequenced (an SphI fragment of 1363 bp) is indicated with a double arrow. The ATG initiation codon and TAA stop codon are also marked.

Hydrolase 1 has excellent hydrolytic activity and can be used to produce peracid from a suitable substrate (for example, a triglyceride such as trioctanoin) in the presence of a peroxide source. It can produce peracid even in the presence of anionic surfactants, which typically inhibit the activity of enzymes.

When produced by fermentation of the P. mendocino strain, then Hydrolase 1 preferably is separated from other proteins and purified by means known to the art, such as by ion exchange and gel permeation chromatography, to yield substantially enzymatically pure Hydrolase 1. This is primarily because the crude fermentation broth of P. mendocino was found to include another enzyme (hereinafter "Hydrolase 2") in addition to Hydrolase 1.

Hydrolase 1 and Hydrolase 2 may be separated by means known to the art such as chromatography. They can be distinguished by their different hydrolysis rates for p-nitrophenyl butyrate and p-nitrophenyl

Hydrolase 1 preferably is produced by cloning to express this enzyme through a host organism, such as a bacteria, yeast, or fungi by techniques known to those skilled in the art. Especially preferred is cloning in E. coli, followed by column chromatography of the cloned Hydrolase 1, as is more particularly described hereinafter. Production by cloning in accordance with the invention provides surprising high yields. Thus, the yield recoverable from fermentation broth of E. coli strain JM101 harboring the plasmid pSNtacII, as described in Example 9, has been found to be up to about 5.5g/liter, with about 3.4g/liter being an average yield. These yields are surprisingly high, since conventional amounts of peptide recovery from E. coli fermentation are on the order of about 0.2-0.3 g/liter. That is, practice of the inventive method can provide about ten times greater yield of the novel hydrolase than could have generally been expected.

Hydrolase 2 is also novel, hydrolzyes glyceride substrates, and may be used in applications such as in fats and oils processing as a digestive aid.

The following experimental methods, materials and results are described for purposes of illustrating the present invention. However, other aspects, advantages and modifications within the scope of the invention will be apparent to those skilled in the art to which the invention pertains.

Experimental

Example 1

(A) Seeding and Fermenting

A seed medium was prepared with 0.6% nutrient broth (Difco) and 1% glucose (pH 6.5). 100 ml of this medium was sterilized in 500 ml fernbach flasks. The flasks were each seeded with a loopful from an overnight culture of P. mendocino ATCC 53552 grown on nutrient agar, and placed on a Newbrunswick shaker at 250 rpm, 37°C for 12 hours. The incubated 12-hour culture was then seeded at appropriate volumes (1-10% v/v) into a 1 liter fermenter (250 ml working volume), a 15 liter Biolafitte fermenter (12 liters working volume), or a 100 liter Biolafitte fermenter provided with a temperature controller, RPM, airflow and pressure controller. The fermenter medium contained 0.6% nutrient broth (Difco), 0.3% apple cutin, and 0.2% yeast extract (Difco), with an initial pH of 6.5. The medium was adjusted to pH 6.8 and sterilized for 40 minutes before seeding. Bacterial growth and enzyme production were allowed to continue in the fermenter for 12-15 hours.

(B) Enzyme Recovery by Microfiltration

The crude fermentation culture was first filtered in a Amicon unit outfitted with two Romicon microporous membranes (0.22u) to remove cells. Remaining enzyme in the retentate which was bound to the cutin particles was removed by centrifugation. Total recovery approached 90%.

(C) Concentration and Dialysis of Whole Cell Filtrate

The recovered filtrate from the Amicon unit was concentrated to a volume of 3 liters on an Amicon ultrafiltration unit with two Romicon Pm 10 modules. The concentrated material was then dialised with 20 liters of 0.01M phosphate buffer, pH 7.5, to remove salts and color. Recovery at this stage averaged about 80%. Total activity for this crude preparation was $8.68 \times 10^6$ units. A unit of hydrolase activity is defined as the amount of enzyme which results in an increase of absorbance at 415 nm of 1.0/minute when incubated at 25°C with 2.0 mM p-nitrophenylbutyrate in 0.1 M pH 8.0 Tris-HCl buffer containing 0.1 wt. % Triton X-100.

Example 2

Hydrolase Activity After Ultrafiltration and Diafiltration

The binding of three p-nitrophenyl substrates and the turnover kinetics were studied for the crude preparation of Example 1(C), where reaction conditions were 0.1M Tris with 0.1 wt. % Triton X-100, pH 8.0, at 25°C. The substrates were p-nitrophenyl caprylate, p-nitrophenyl laurate, and p-nitrophenyl palmitate, and the data is set out in Table 1.

Table 1

| Substrate | $K_m(\mu M)$ | $V_{max}(\mu mole/min/mg\ protein)$ |
|-----------|--------------|-------------------------------------|
| PNPC | 214 | 802 |
| PNPL | 167 | 214 |
| PNPP | 183 | 112 |

The Example 1(C) preparation was used in a variety of experiments; however, the Example 1(C) preparation includes two enzymes designated "Hydrolase 1" and "Hydrolase 2". Hydrolase 1 is the better perhydrolase. A separation and purification of the crude Example 1(C) preparation is described in Example 3, a complete separation of Hydrolase 1 and Hydrolase 2 is described in Example 4 (preferred to obtain substantially enzymatically pure Hydrolase 1), and an extremely pure sample of Hydrolase 1 preparation (i.e. analytically pure for sequencing) is described in Example 5.

Example 3

Partial Purification of Hydrolase 1 and Hydrolase 2 by Ion Exchange and Gel Permeation Chromatography

Hydrolase 1 was initially partially purified from the Pseudomonas mendocino fermentation broth by DEAE Sephacryl chromatography followed by Sephadex G-100 gel permeation chromatography. A DEAE column was equilibrated in 10 mM sodium phosphate buffer, pH 8, and the crude protein was applied to the column in the same buffer. PNB (p-nitrophenyl butyrate) hydrolase activity that was not bound to the column was associated with Hydrolase 1. Hydrolase 1 thus obtained from the DEAE step was subjected to chromatography on Sephadex G-100 in 10 mM sodium phosphate buffer pH 8. Hydrolase 1 eluted from this column as a discrete peak, and was identified by PNB hydrolase activity as well as perhydrolytic activity.

Example 4

Complete Separation of Hydrolase 1 and Hydrolase 2 by Hydrophobic Chromatography

Hydrolase 1 may be separated completely from Hydrolase 2 by chromatography on hydrophobic resins. The enzyme solution of Example 1(C) after ultrafiltration and diafiltration was adjusted to 0.5M NaCl and applied to a 0.8 x 7 cm octyl Sepharose column equilibrated in 10mM Tris(Cl) pH 8, 0.5M NaCl and washed to remove unbound protein. The following washes were then employed: 10mM Tris(Cl), pH 8, 2M urea; 10mM Na phosphate pH 8; 10mM phosphate, pH 8, 0.5M NaCl. After washing, the column was then developed with a linear gradient to 50% n-propanol. The column fractions were then assayed for activity on p-nitrophenyl butyrate (PNB) and p-nitrophenyl caprylate (PNC) in order to locate the enzymatic activities. Two enzymes were clearly resolved, fraction 32 with a PNB/PNC ratio of 4.6 and fraction 51 with a PNB/PNC ratio of 1.40. These have been designated Hydrolase 1 and Hydrolase 1, respectively.

The fractions from this column were further analyzed by SDS gel electrophoresis. This analysis revealed that the two enzyme activities track with 30,000 molecular weight bands characteristic of procaryotic enzymes; in addition, Hydrolase 2 migrated as a doublet, and was clearly resolved from the single band of Hydrolase 1. Prior to sequence analysis, these two partially purified enzymes were separated from the high and low molecular weight contaminants by reverse phase chromatography.

6

Example 5

Purification of Hydrolase 1 by HPLC in Preparation for Enzyme Peptide Fragmentation

Prior to sequence analysis, the partially purified material of Example 3 was further purified by chromatography on a 4.8 x 100 mm, SynChromPak C4 reverse phase HPLC column. The system was equilibrated in 0.05% triethylamine (TEA) and 0.05% trifluoroacetic acid (TFA) (Solvent A) at 0.5 mL/min. 100$\mu$g to 1 mg of Hydrolase 1 was injected onto the column and the protein eluted by a compound gradient of Solvent A and n-propanol containing 0.05% and 0.05% TFA (Solvent B). A typical gradient was +5% from 0 to 20% B and then +0.5% B/minute to 60% B. All enzyme is inactivated by this HPLC solvent system. The protein peaks eluting at about 35% solvent B (Hydrolase 1) or at about 39% Solvent B (Hydrolase 2) were collected and used for further sequence analysis and preparation of CNBr fragments.

Example 6

Preparation and Purification of Cyanogen Bromide Peptide Fragments for Amino Acid Analysis

The cyanogen bromide peptide fragments for amino acid sequence analysis were prepared and purified as follows. An aliquot of pooled Hydrolase 1 of Example 5 was dried in a SpeedVac centrifuge and then resuspended to 10 mg/ml in 8 M urea, 88% formic acid. The solution was mixed with one volume of 200 mg/ml CNBr in formic acid and incubated in the dark at room temperature for 2 hours. The product was then desalted into 40% solvent B:50% solvent A (see above) on a 0.8 x 7 cm IBF-TrisAcryl GF05 (coarse) column prior to reverse phase analysis. The peptides were initially separated using the same protocol as listed above for the purification of Hydrolase 1 by reverse phase. Solvent B, however, was changed to 35% propanol:65% acetonitrile (containing TEA and TFA). The initial digest and the peaks after chromatography were also analyzed on SDS/urea/pyridine gels followed by silver staining.

Two peaks were chosen from the chromatogram and subjected to rechromatography employing the conditions dictated above, this time on a 0.48 x 25 cm SynChromPak C4 column. After rechromatography, the purified peptides were held for sequence analysis.

Example 7

Distinction of Hydrolase 1 from Hydrolase 2: Preparation of Cyanogen Bromide Fragments of Hydrolase 1 and Hydrolase 2

The purified fractions of Hydrolase 1 and Hydrolase 2 from the octyl Sepharose column (as in Example 4) were each diluted with 3 volumes of solvent A (0.05% triethylamine and 0.05% trifluoroacetic acid) and chromatographed (as in Example 5). As described in Example 4, the purified proteins were analyzed by SDS gel electrophoresis, and then pooled individually for comparison of the CNBr fragments and the N-terminal amino acid sequences of Hydrolase 1 and Hydrolase 2.

Example 8

Specific Activity of Hydrolase 1

The specific activity of Hydrolase 1 was determined using the enzyme purified as in Example 4. Substantially enzymatically pure Hydrolase 1 has a specific enzyme activity of 3750 units per mg protein as defined in Example 1(C).

Example 9

Preparation of Cloned Hydrolase 1 in E. Coli Cloning of the Hydrolase 1 Gene of Pseudomonas mendocino

The Pseudomonas mendocino strain (ATCC 53552) was grown overnight at 37°C in 200 ml LB (Luria Broth) medium. Cells were harvested by centrifugation and high molecular weight total DNA was prepared exactly according to a standard procedure as outlined by Birnboim et al., Nucleic Acids Res. 7, pp. 1513-1523 (1979). The DNA was digested to completion with EcoRI and ligated with T4 DNA ligase to a preparation of plasmid pBR322 (ATCC 37017) digested with EcoRI and dephosphorylated with bacterial alkaline phosphatase. All enzymes used for the manipulation of DNA were used according to the manufacturers' directions (New England Biolabs or Bethesda Research Laboratories). The ligated DNA was used to transform E. coli 294 (ATCC 31445) and ampicillin resistant (Ampr) colonies were selected. Accordingly, approximately $2 \times 10^4$ transformants were obtained (approximately $5 \times 10^3$/plate). Plates were flooded with a solution of 4-methylumbelliferylbutyrate (10mM in 50 mM Tris-HCl, pH 8.0) and then illuminated with an ultraviolet lamp (wavelength 340 nm). Colonies which hydrolyzed the substrate to release the highly fluorogenic compound 4-melthylumbelliferone appeared as intensely blue. Using this method 13 positive colonies were obtained. From each of these positive colonies a plasmid miniprep was prepared by the alkaline lysis method as described in Birnboim, supra. Each plasmid was digested with EcoRI and resulting fragments were resolved by polyacrylamide gel electrophoresis as described by Maniatis et al., Molecular Cloning: A Laboratory Manual, Cold Spring Harbor Laboratory, Cold Spring, New York (1982). Most plasmids contained a single inserted fragment of 4.3 kb. The others contained other fragments in addition to this fragment. This result suggested that all positive colonies arose as a result of the expression of a common cloned gene contained on the 4.3 kb fragment. One of the plasmids which contained only the 4.3 kb fragment, designated pSNE4, was selected for detailed analysis.

Plasmid pSNE4 was digested with a variety of restriction enzymes which have 6 bp recognition sequences. These enzymes were used singly or in pairs. Analysis of the fragment sizes resulting from these experiments allowed the generation of a preliminary restriction endonuclease cleavage map of the 4.3 kb EcoRI insert of PSNE4. This map is shown in Figure 1.

Several subfragments of the EcoRI insert of plasmid PSNE4 which were at least 840 bp were subcloned into pBR322 in order to see if any contained a functional gene. Among the plasmids which were found to contain functional hydrolase genes was pSNES1, which contains a 2.3 kb EcoRI/SalI fragment from the EcoRI insert of pSNE4. (See Figure 1 for map location of this fragment).

The inserted fragment of pSNES1 was digested with further restriction enzymes and the resulting small fragments were subcloned into bacteriophage M13 vectors, described by Roberts, Nucleic Acids Res., 12, supplement r167-r204 (1984), for sequencing by the dideoxy chain termination method of Sanger et al., Proc. Natl. Acad. Sci. USA 74, pp. 5463-5467 (1977). The sequence of the 1.36 kb of DNA between the SphI sites (refer to Figure 1), when translated in all possible reading frames, revealed a large open reading frame which includes the $NH_2$-terminal amino acid residues of the protein as determined by direct amino acid sequencing (residues 1-16). This open reading frame also contains the code for two other directly sequenced peptides (residues 94-105 and residues 173-190). The methionine at position -22 is believed to be the initiation codon because it begins the code for a highly hydrophobic region typical of signal peptides. This signal peptide is presumably cleaved off during the secretion process after the alanine at position -1. The open reading frame ends at position 259, indicating that the encoded mature protein has 258 residues.

Regulated Expression of
P. mendocino Hydrolase 1 Gene in E. coli

In order to achieve the regulated expression of the P. mendocino hydrolase gene in E. coli, an XbaI site was first introduced just before the ATG initiation codon by site directed mutagenesis, Adelman et al., DNA 2, pp. 183-193 (1983) in bacteriophage M13, and the modified gene was subsequently cloned into an expression vector which contains the strong tacII promoter, deBoer et al., Proc. Natl. Acad. Sci. USA 80, p. 2125 (1983). This was done by first digesting pSNES1 with SphI.

The 2.4 kb SphI fragment containing the entire hydrolase coding sequence was isolated and ligated into the replicative form (RF) of M13mp19 at its SphI site and the mixture was used to transfect E. coli JM101 (ATCC 33876). Clear plaques were picked and the bacteriophage (template) DNA in which the SphI fragment was present in a counterclockwise orientation was prepared. A partially complementary single-stranded fragment of DNA consisting of 50 nucleotides was synthesized which contained an XbaI site immediately 5′ of the Hydrolase 1 ATG initiation codon. This 50-mer complements the template DNA from the -27 nucleotide position (before the ATG intiation codon) to the -9 position and from the +1 (the A of the ATG) to the +20 position. Between the -9 and the +1 positions, however, the sequence 5′-AACCTTCG-3′ of the native hydrolase promoter region was to be changed to 5′-TATCTAGAATT-3′ of the tacII promoter. Mutagenesis was performed.

Three hundred plaques were screened by hybridization with a $^{32}$P-labeled synthetic oligonucleotide (5′-ATGAGGTATCTAGAATTATG-3′) which spans the area of change. An RF of a positively hybridizing clone was prepared and cleaved with XbaI and SphI. A 1 kb XbaI/SphI fragment containing the gene was isolated and ligated into a vector obtained by digesting pHGH907tacII, described by deBoer, supra, with XbaI and SphI and isolating a 4.1 kb XbaI/SphI fragment containing the tacII promoter, and the ampicillin resistance gene. JM101 cells were then transformed with the ligation mixture. An ampicillin resistant colony (containing plasmid pSNtacII--see Figure 2) was selected.

To determine the levels of cloned Hydrolase 1 synthesized by E. coli, JM1O1/psNtacII was grown in 20 mls LB medium supplement with 1mM isopropyl-B-D-thiogalactoside (IPTG) for 10h at 37°C. 294/pBR322 was used as a negative control. The cells were separated from the culture supernatant by centrifugation and then fractionated into periplasmic and membrane/cytoplasmic components, Koshland, supra. Each fraction was tested for activity by p-nitrophenylbutyrate hydrolysis. $\beta$-lactamase (periplasmic marker) and $\beta$-galactosase (cytoplasmic marker) were also measured, Gray et al., Proc. Natl. Acad. Sci. USA 81, pp. 2645-2649 (1984), in order to confirm the effectiveness of the cell fractionation procedure.

Most of the Hydrolase 1 activity (74%) was present in the culture supernatant. Most of the cell associated enzyme was found in the cell wash fraction (17% of the total) with smaller amounts present in the periplasmic (2%) and cytoplasm/membrane (7%) fractions. No Hydrolase 1 activity was present in any fractions of the 294/pBR322 negative control culture. Yields of Hydrolase 1 in eight fermentations as just described (10 liter fermenters) were between 1.5 g/liter and 5.5 g/liter, for an average yield of 3.4 g/liter.

Broth from the fermentation of E. coli strain JM101 harboring the plasmid pSNtacII was adjusted to 0.5M NaCl and purified by octyl Sepharose substantially as described when P. mendocino fermented (Example 4), except the propanol gradient was eliminated and elution was achieved with 20% acetonitrile in 10mM Na phosphate, pH 8, 0.5M NaCl. The isolated product (cloned from the gene expressing the enzyme) was analyzed by SDS gels and migrated identically to the Hydrolase 1 product isolated from the original Pseudomonas mendocino strain.

Example 10

Preparation of Cyanogen Bromide Fragments from Cloned Hydrolase 1

Cyanogen bromide fragments from cloned Hydrolase 1 were prepared as follows. The product from the octyl Sepharose purification of cloned product (Example 9) was diluted with 3 volumes of solvent A and purified on the short C4 HPLC column, as described for Hydrolase 1 and Hydrolase 2 isolated from Pseudomonas mendocino. The product was analyzed on SDS gel.

Example 11

Comparison of CNBr Fragments of Hydrolase 1 from P. putida and CNBr Fragments from the Cloned Hydrolase 1 in E. coli

CNBr fragments of Hydrolase 1 from P. mendocino and CNBr fragments from the cloned Hydrolase 1 in E. coli were compared. HPLC purified Hydrolase 1 and 2 from Pseudomonas and the cloned Hydrolase 1 were each hydrolyzed by CNBr as described in Example 6 above. The products were analyzed by SDS/urea/pyridine electrophoresis. The results indicate the cloned protein is clearly Hydrolase 1. Hydrolase 1 isolated from P. mendocino (as in Examples 4-5) was shown to be identical to the cloned Hydrolase 1 isolated from E. coli by the following criteria: (a) Hydrolase 1 from either organism was isolated by the same chromatographic procedure (as in Example 4); (b) the amino acid sequences of the N-terminal of the Hydrolase 1 isolated from either organism were the same; (c) the CNBr fragment pattern showed that the Hydrolase 1 and Hydrolase 2 are clearly distinguished and that the CNBr fragments of Hydrolase 1 from either P. mendocino or E. coli are identical; (d) the p-nitrophenyl butyrate and p-nitrophenylcaprylate substrate activity ratio of Hydrolase 1 from both bacterial sources is the same; and (e) the hydrolysis/perhydrolysis ratio with tricaprylin as substrate is the same for Hydrolase 1 as isolated from both organisms.

When separated, Hydrolase 1 and Hydrolase 2 were found to have quite different hydrolysis rates (hydrolytic activity) for p-nitrophenyl butyrate and for p-nitrophenyl caprylate. Thus, the two novel enzymes can be distinguished by their ratios of p-nitrophenyl butyrate to p-nitrophenyl caprylate hydrolysis, as illustrated by Example 12.

Example 12

Hydrolysis Rates of Hydrolase 1 and Hydrolase 2 with p-Nitrophenyl Butyrate and p-Nitrophenyl Caprylate as Substrates

The reactions were performed in samples containing 0.1 M Tris HCl, pH 8.0 with 0.1 wt. % Triton X-100 nonionic surfactant (available from Rohm & Haas) at 25°C. The hydrolysis rates of 2.0 mM p-nitrophenyl butyrate (PNB) for Hydrolase 1 (as from Example 3), was 0.60 ( OD 415 nm/min.), while that of 2.0 mM p-nitrophenyl caprylate (PNC) was 0.09, for a PNB/PNC ratio of 7. By contrast, the hydrolysis rate of PNB for Hydrolase 2 at the same concentration was 0.54, of PNC at the same concentration was 0.44, for a PNB/PNC ratio of 1.

Example 13 illustrates stain removal studies using Hydrolase 1.

Example 13

Diagnostic evaluations of oxidant performance were performed with 100% cotton swatches stained with crystal violet as follows. Crystal violet (0.125 g) was added to 1.25 liters of distilled water. One hundred two-inch by two-inch undyed, 100% cotton swatches were added to the solution and agitated for eight hours. The cotton swatches (not dyed with crystal violet) were removed from the staining solution and rinsed repeatedly with cold tap water until the effluent was nearly clear. The stained swatches were then individually placed on aluminum foil, blotted with paper towels, and allowed to air dry.

A fomulation utilizing Hydrolase 1 was prepared, as was a corresponding control composition. Both compositions were each used to wash the stained cotton swatches and the stain removal performance evaluated for each. The performance results are summarized in Table 2.

## Table 2

| Composition with Hydrolase 1 | |
| --- | --- |
| 0.06 wt. % trioctanoin | 80.4 |
| 0.04 wt. % sodium dodecylsulfate | |
| 200 ppm $H_2O_2$ | |
| 1 µg/ml Hydrolase 1 | |
| 20 µM EDTA | |
| (pH = 10.5) | |

| Control Composition | |
| --- | --- |
| 0.06 wt. % trioctanoin | 69.8 |
| 0.04 wt. % sodium dodecylsulfate | |
| 200 ppm $H_2O_2$ | |
| 20 µM EDTA | |
| (pH = 10.5) | |

As may be seen from the data of Table 2, the composition including Hydrolase 1 provided improved stain removal benefits with respect to the control composition even though the control composition included the hydrogen peroxide component. This improved stain removal was particularly striking as occurring in the presence of anionic surfactant which inhibits many prior known commercially available enzymes.

While the invention has been described in connection with specific embodiments thereof, it will be understood that variations in the product and the method of obtaining it will be available to those skilled in the art on the basis of this disclosure. For example, as indicated above, the P. mendocino microorganism source of the hydrolase or its DNA is capable of mutation, by conventional mutagenesis procedures and/or selective pressures, to produce strains which have a higher output of the hydrolase or which produce a mutant hydrolase of equivalent or better properties. The present invention therefore includes methods of preparing the hydrolase using such mutants and variants in the hydrolase which retain the essential properties of the enzyme, and in particular a hydrolysis rate which is at least as great as for the specific hydrolase 1 described herein. By the hydrolysis rate test of Example 12 above, this would indicate a PNB/PNC ratio of at least 7.

Variations in the method of making the hydrolase include the possibility of total protein synthesis, but more probably the production of the protein by expression in a recombinant host. For this purpose, the encoding DNA may be identified in the source microorganism using specific oligonucleotide hybridisation probes, for example synthesised according to the DNA sequence given herein. Alternatively, or additionally, the encoding DNA or any part of it, whether of the sequence given herein or of a variant permitted by the degeneracy in the genetic code, can be synsthesised rather than extracting it from its natural source.

The production of the hydrolase by genetic engineering techniques permits the amino acid sequence to be varied at will, for example introducing point mutations, or insertions, deletions, additions or other substitutions, so that possibly improved variants in the hydrolase can be prepared and investigated by routine procedures.

All such variations as are mentioned above are included within the scope of the present invention.

## Claims

1. A substantially enzymatically pure hydrolase having the amino acid sequence dipicted herein or a variant thereof having an equivalent or greater hydrolysis rate.

2. A variant hydrolase of claim 1 having a hydrolysis rate at least as great as that of the hydrolase of the amino acid sequence depicted herein, as measured by the ratio (the PNB/PNC ratio) of the rates of hydrolysis of equimolar concentrations of p-nitrophenyl butyrate (PNB) and p-nitrophenyl caprylate (PNC).

3. A variant hydrolase of claim 2 wherein the PNB/PNC ratio, measured at 2.0 mM concentration in 0.1M Tris HCl.pH 8.0 with 0.1wt% non-ionic surfactant, at 25$^\circ$C, is at least 7.

4. A method for producing the hydrolase of any one of claims 1, 2 and 3, comprising:
   culturing a recombinant microorganism transformed with a recombinant expression vector including a gene which expresses the hydrolase, to express the gene for the hydrolase; and
   isolating and purifying said expressed hydrolase.

5. A method according to claim 4 wherein the microorganism is a bacterium.

6. The method as in claim 5 wherein the bacterial transformants are E.coli.

7. The method as in any one of claims 4, 5 and 6 wherein purification of the expressed hydrolase includes column chromatography.

8. The method which comprises extracting from Pseudomonas mendocino (strain ATCC 53552) or a mutant thereof a substantially enzymatically pure hydrolase having a specific enzyme activity of at least about 3750 units per mg protein, wherein a unit is the amount of enzyme which results in an increase of absorbance at 415 nm of 1.0/minute when the enzyme is incubated at 25$^\circ$C with 2.0 mM p-nitrophenyl butyrate in 0.1 M pH 8.0 Tris-HCl buffer containing 0.1 wt.% octylphenol ethoxylate nonionic surfactant, HLB 13.5.

9. Pseudomonas mendocino (strain ATCC 53552), or a mutant thereof capable of producing a hydrolase of any one of claims 1 to 3 and 8.

10. A recombinant microorganism according to any one of claims 4,5, and 6.

## Revendications

1. Hydrolase sensiblement pure sur le plan enzymatique possédant la séquence aminoacide décrite présentement ou une variante de celle-ci possédant une vitesse d'hydrolyse plus grande ou équivalente.

2. Hydrolase variante suivant la revendication 1 possédant une vitesse d'hydrolyse au moins aussi grande

EP 0 268 452 B1

que celle de l'hydrolase de la séquence aminoacide décrite présentement, lorsque mesurée par le rapport (rapport PNB/PNC) des vitesses d'hydrolyse de concentrations équimolaires du p-nitrophényl butyrate (PNB) et du p-nitrophényl caprylate (PNC).

3. Hydrolase variante suivant la revendication 2, dans laquelle le rapport PNB/PNC, mesuré à concentration 2 mM dans Tris HCl 0,1M, pH 8 avec un surfactant non ionique 0,1% en poids, à 25 °C est d'au moins 7.

4. Procédé pour produire l'hydrolase suivant l'une quelconque des revendications 1, 2 et 3, consistant à :
   cultiver un microorganisme recombinant transformé avec un vecteur d'expression recombinant comprenant un gène qui exprime l'hydrolase, pour exprimer le gène pour l'hydrolase ; et
   isoler et purifier ladite hydrolase exprimée.

5. Procédé suivant la revendication 4, dans lequel le microorganisme est une bactérie.

6. Procédé suivant la revendication 5, dans lequel les transformants bactériens sont E. coli.

7. Procédé suivant l'une quelconque des revendications 4, 5 et 6, dans lequel la purification de l'hydrolase exprimée est effectuée par chromatographie en colonne.

8. Procédé qui consiste à extraire de Pseudomonas mendocino (souche ATCC 53552) ou un mutant de celle-ci une hydrolase sensiblement pure sur le plan enzymatique possédant une activité d'enzyme spécifique d'au moins environ 3750 unités par mg de protéine, où une unité est la quantité d'enzyme qui provoque une augmentation d'absorption à 415 nm de 1/minute lorsque l'enzyme est incubé à 25 °C avec 2 mM de p-nitrophényl butyrate dans du tampon Tris-HCl 0,1 M, pH 8, contenant 0,1% en poids de surfactant non ionique d'étoxylate octylphénol, HLB 13,5.

9. Pseudomonas mendocino (souche ATCC 53552), ou un mutant de celle-ci capable de produire une hydrolase suivant l'une quelconque des revendications 1 à 3 et 8.

10. Microorganisme recombinant suivant l'une quelconque des revendications 4, 5 et 6.


**Patentansprüche**

1. Im wesentlichen enzymatisch reine Hydrolase, enthaltend eine Aminosäuresequenz oder eine Variante davon mit einer äquivalenten oder höheren Hydrolysenrate.

2. Hydrolase-Variante nach Anspruch 1 mit einer Hydrolysenrate, die mindestens ebenso hoch ist wie diejenige der Hydrolase mit der Aminosäuresequenz darin, gemessen durch das Verhältnis zwischen den Hydrolysenraten von äquimolaren Konzentrationen an p-Nitrophenylbutyrat (PNB) und p-Nitrophenylcaprylat (PNC) (PNB/PNC-Verhältnis).

3. Hydrolase-Variante nach Anspruch 2, worin das PNB/PNC-Verhältnis, gemessen bei einer Konzentration von 2,0 mM in 0,1 M Tris-HCl · pH 8,0 mit 0,1 Gew.-% eines nicht-ionischen Tensids bei 25 °C, mindestens 7 beträgt.

4. Verfahren zur Herstellung der Hydrolase nach einem der Ansprüche 1, 2 und 3, das umfaßt

   die Kultivierung eines Rekombinanten-Mikroorganismus, der transformiert worden ist mit einem Rekombinanten-Expressionsvektor, der ein Gen enthält, welches die Hydrolase exprimiert, um das Gen für die Hydrolase zu exprimieren; und

   das Isolieren und Reinigen der exprimierten Hydrolase.

5. Verfahren nach Anspruch 4, worin der Mikroorganismus ein Bakterium ist.

6. Verfahren nach Anspruch 5, worin die bakteriellen Transformanden E. coli sind.

7. Verfahren nach einem der Ansprüche 4, 5 und 6, worin die Reinigung der exprimierten Hydrolase eine Säulenchromatographie umfaßt.

8. Verfahren, das umfaßt das Extrahieren einer im wesentlichen enzymatisch reinen Hydrolase mit einer spezifischen Enzymaktivität von mindestens etwa 3 750 Einheiten pro mg Protein aus Pseudomonas mendocino (Stamm ATCC 53552) oder einer Mutanten davon, wobei eine Einheit die Enzymmenge ist, die zu einer Erhöhung der Extinktion bei 415 nm von 1,0/min führt, wenn das Enzym mit 2,0 mM p-Nitrophenylbutyrat in einem 0,1 M pH 8,0 Tris-HCl-Puffer, enthaltend 0,1 Gew.-% nicht-ionisches Octylphenolethoxylat-Tensid mit HLB 13,5 bei 25°C inkubiert wird.

9. Pseudomonas mendocino (Stamm ATCC 53552) oder eine Mutante davon, das (die) in der Lage ist, eine Hydrolase nach einem der Ansprüche 1 bis 3 und 8 zu bilden.

10. Rekombinanten-Mikroorganismus nach einem der Ansprüche 4, 5 und 6.

Fig 1

Fig 2